# EUROPEAN PATENT APPLICATION

(11) **EP 1 930 018 A1**
(43) Date of publication of application: **11.06.2008**
(21) Application number: 07022928.1
(22) Date of filing: 27.11.2007
(51) Int. Cl.: A61K 35/74, C12N 1/20

(54) **Strains of probiotic lactobacilli that persist in the intestine and modulate its mucosal receptors**

(30) Priority: 06.12.2006 IT MI20062363
(71) Applicant: ATT - Advanced Analytical Technologies SRL, 29100 Piacenza (IT)
(72) Inventor: Elli, Marina, 29100 Piacenza (IT); Bessi, Elena, 29100 Piacenza (IT); Cattivelli, Daniela, 29100 Piacenza (IT); Soldi, Sara, 29100 Piacenza (IT); Morelli, Lorenzo, 29100 Piacenza (IT)
(74) Representative: Minoja, Fabrizio

(57) **Abstract**

Disclosed are strains of *Lactobacillus crispatus* able to stimulate the host immune system by producing hydrogen peroxide and consequently modulating receptor PPAR-γ.

## Description

The present invention relates to strains of *Lactobacillus* which interact with the immune system of the human and/or animal host, causing the expression of toll-like receptors TLR2 and TLR4 which are responsible for inducing the immune response at mucosal level.

The invention also relates to probiotic compositions containing the strains according to the invention.

### Background to the invention

Although numerous articles relating to stimulation of the human or animal host immune system by probiotic bacteria have been published, the action mechanism is still unknown. Moreover, not all bacterial strains perform probiotic actions.

The epithelial cells of the intestinal mucosa are no longer considered to be no more than a mechanical barrier against invasion by pathogenic bacteria; it has been demonstrated that they receive a series of environmental stimuli and activate response mechanisms in order to maintain intestinal homeostasis, suppress pathogens and react to the ingestion of probiotics.

The definition "bacteria-host interaction" was recently coined to describe the specific case of the relations between the intestinal microflora and the mucosa. The mucosal immune and non-immune cells are able to recognise microbial products, which are often located on the surface of bacterial cells, such as the lipopolysaccharides (LPS) of Gram-negative bacteria and the peptidoglycan and lipoteichoic acid (LTA) of Gram-positive bacteria.

Said recognition takes place through receptors, including Toll-like Receptors (TLRs). The TLRs include TLR4, which recognises and binds the LPS of Gram-negative bacteria, and TLR2, which binds the LTA of Gram-positive bacteria.

When stimulated by bacterial molecules, the TLRs, through cofactors, activate transcription factors that modulate the expression of proinflammatory and anti-inflammatory cytokines and chemokines which, in turn, regulate the activity of the immune system.

Peroxisome proliferator-activated receptor-gamma (PPARgamma) is a nuclear receptor highly expressed in the colon, which plays a key part in regulating inflammation of the colon induced by micro-organisms.

### Description of the invention

It has now been found, and this is the object of the invention, that strains of *L. crispatus* stimulate the host immune system, modulating the PPAR-y receptor by producing hydrogen peroxide, according to a mechanism which has never before been described. The strains according to the invention have properties of intestinal persistence, measured by evaluating their ability to adhere to the intestinal mucosa of mice and to remain viable in their faeces.

The invention relates in particular to the strain *L. crispatus* M247, identified in the faeces and deposited under access number LMG P-23257 dated 11 April 2006 in the BCCM/LMG Bacteria Collection, Laboratorium voor Microbiologie, Ghent University, K.L. Ledeganckstraat 35, B-9000 Ghent, Belgium, pursuant to the Treaty of Budapest. A comparator strain of *L. crispatus,* identified by the code MU5, was deposited in the same collection on the same date under access number LMG P-23258.

Comparison of the different effects induced by the two deposited strains on the immune system explains the mechanism used by strain M247 to induce the immune response in the host.

In particular, the effects of the two strains MU5 and M247 on the host immune system were compared by the following procedure:
a) Analysis and selection of lactobacilli that stimulate the host immune system;
b) Analysis and selection of aggregating probiotic lactobacilli able to persist in the host intestine, and their spontaneous non-aggregating isogenic mutants;
c) Analysis and selection of probiotic lactobacilli that interact with the mucosal receptors;
d) Analysis and selection of lactobacilli that induce activation of the TLR receptors;
e) Analysis and selection of lactobacilli that modulate the PPARgamma receptor;
f) Analysis and selection of lactobacilli that modulate the PPARgamma receptor by producing hydrogen peroxide.

The results obtained, reported in the Examples below, demonstrate the advantageous properties of the strains according to the invention, especially *L. crispatus* M247, which could usefully be employed in the fields of human and veterinary medicine and diet.

For this purpose, the strains according to the invention could be administered in suitable probiotic formulations containing an effective number of live or freeze-dried cells. Examples of these formulations include capsules, suspensions in water or other liquids, tablets, granulates, powders, topical formulations for application to the mucous membranes, e.g. creams, ointments, lotions, spray; and dietary formulations e.g. for use as food supplements and the like.

### EXAMPLE 1

### Persistence of L. crispatus M247 and L. crispatus MU5 in the faeces and on the tissues of treated mice

The test was carried out on 3 groups of mice, each consisting of 12-20 mice, which were administered with 100 µl of suspension daily for 14 days.

The suspension contained saline in the case of control group (C), and 100,000,000 bacterial cells in the case of the group treated with *L. crispatus* M247 (M247) or *L. crispatus* MU5 (MU5).

Immediately before the start (T0) and at the end of the administration period (T14), stool samples were taken from mice C, M247 and MU5.

The animals were sacrificed at the end of the administration period and the proximal portion of the colon was removed and divided into several parts, which were treated differently and stored for subsequent applications, such as determination of the total lactobacilli and extraction of RNA.

The stool samples and tissue samples were used to determine the presence of bacterial strains *L. crispatus* M247 and *L. crispatus* MU5 in the treated groups, by strain-specific PCR.

The tissue samples were also used to recover the epithelial cells and extract messenger RNA to be used as a template in the RT-PCR tests.

This technique was used to evaluate the level of transcription of interleukin-6 (IL-6), interleukin-10 (IL-10) and toll-like receptors (TLR2 and TLR4).

The strains *L. crispatus* M247 and *L. crispatus* MU5 were not present in the faeces of the treated mice before the start of the test.

After 14 day administration of the bacterial suspension, strain *L. crispatus* M247 was found in the faeces of 12 of the 17 mice in group M247, whereas strain *L. crispatus* MU5 was only found in the faeces of 2 of the 14 mice in group MU5.

It can therefore be concluded that strain *L. crispatus* M247 is capable of surviving much better in the intestine of the treated mice than strain *L. crispatus* MU5.

After 14 day administration of the bacterial suspension, strain *L. crispatus* M247 was found in the tissue samples of 9 of the 17 mice in group M247, while strain *L. crispatus* MU5 was only found in the colon of 5 of the 14 mice in group MU5.

This result also demonstrates that strain *L. crispatus* M247 is capable of surviving much better than strain *L. crispatus* MU5 in the intestine of the treated mice.

Tables 1, 2 and 3 show the data relating to said results.

**Table 1. Number of mice in which strains L. crispatus M247 and L. crispatus MU5 were identified, out of the total of treated mice.**

| | Faeces | Tissue |
|---|---|---|
| | T14 | T14 |
| M247 treated Mice | 12/17 | 9/17 |
| MU5 treated Mice | 2/14 | 5/14 |

**Table 2. Identification of L. crispatus M247 and L. crispatus MU5 in the faeces and tissues of the treated mice.**

| No. of identifications per mouse¹ | *L. crispatus M247* | | *L. crispatus MU5* | |
|---|---|---|---|---|
| | No. of mice whose faeces contained the live probiotic | Mean log₁₀ of CFU/g of wet sample | No. of mice whose faeces contained the live probiotic | Mean log₁₀ of CFU/g of wet sample |
| **Faeces** | | | | |
| Twice | 3 | 7.30 ± 0.44 | 0 | N/I |
| Once | 9 | 8.22 ± 0.25 | 2 | 8.67 ± 0.05 |
| 0 times | 5 | N/I² | 12 | N/I |
| **Tissues** | | | | |
| Once | 9 | 7.09 ± 0.21 | 5 | 7.24 ± 0.64 |
| 0 times | 8 | N/I | 9 | N/I |
| | | | | |
| Total no. of mice | 17 | | 14 | |

| | | | | |
|---|---|---|---|---|
| ¹ "Twice" refers to the identification of *L. crispatus* M247 or *L. crispatus* MU5 at both time T7 and time T14, and "once" indicates that the strains were only identified at time T14. ² N/I = not identified | | | | |

**Table 3. Quantitation of L. crispatus M247 and L. crispatus MU5 in the faeces and tissues of the treated mice (data expressed as log₁₀ CFU).**

| Mouse no. | Faeces | | Tissues | |
|---|---|---|---|---|
| | **No. of live bacteria expressed in log₁₀ CFU** | | | |
| | M247 | MU5 | M247 | MU5 |
| 1 | 9.4 | 8.8 | 7.5 | 8.0 |
| 2 | 8.4 | 8.6 | 6.7 | 8.0 |
| 3 | 8.1 | N/I | 7.1 | 7.5 |
| 4 | 8.8 | N/I | 7.3 | 7.4 |
| 5 | 8.1 | N/I | 7.6 | 5.2 |
| 6 | 8.5 | N/I | 6.8 | N/I |
| 7 | 7.9 | N/I | 7.8 | N/I |
| 8 | 7.8 | N/I | 6.9 | N/I |
| 9 | 6.9 | N/I | 6.1 | N/I |
| 10 | 7.5 | N/I | N/I | N/I |
| 11 | 8.8 | N/I | N/I | N/I |
| 12 | 7.3 | N/I | N/I | N/I |

The presence of strain M247 in the faeces of the mice treated with that probiotic was also confirmed by Denaturing Gradient Gel Electrophoresis (DGGE) carried out on DNA extracted directly from the murine faeces. The method used is based on the findings reported by Konstantinov et al. (2004). The bacterial DNA was extracted from the faeces of the control mice and the mice treated with strains M247 and MU5, and amplified via PCR nested with 2 primer pairs, as indicated by Konstantinov 2004. The amplified samples were subjected to electrophoretic analysis in acrylamide gradient. The highlighted fragments were cut from the gel, eluted and amplified by PCR. The amplification products were sequenced to obtain the taxonomic identification. Strain M247 was identified in the treated mice, whereas strain MU5 was not found. This finding confirms that only the aggregating strain M247 persists in the intestine of mice, and can be found in the faeces of the treated animals.

### EXAMPLE 2

### Persistence of L. crispatus M247 and L. crispatus MU5 in the faeces and on the tissues of patients affected with ulcerative colitis in remission phase

The test was carried out on 1 human female subject of age 48, affected with ulcerative colitis in remission phase. The subject was given the M247 strain in the lyophilized form in a daily dosage of 8 x 10⁹ CFU, for 14 consecutive days.

Immediately before the start (T0), during (T7) and at the end of the administration period (T14), stool samples were taken. Bioptic samples form the colon (n. 4) were taken at T0 and T14.

The stool samples and bioptic samples were used to determine the presence of bacterial strain *L. crispatus* M247 in said samples, by strain-specific PCR.

The strains *L. crispatus* M247 was not present in the faeces and bioptic samples of the subject before the start of the test (T0).

After 14 day administration of the bacterial suspension, strain *L. crispatus* M247 was found in the faeces of the treated subject. It can therefore be concluded that strain *L. crispatus* M247 is capable of surviving in the human intestine after 7 day administration.

After 14 day administration, strain *L. crispatus* M247 was found in the faeces and in 3 out of 4 bioptic samples taken.

It can therefore be concluded that strain *L. crispatus* M247 is capable of surviving in the intestine of the treated subject and of adhering to the colon mucosa.

Table 4 shows the data relating to said results.

**Table 4. Detection of L. crispatus M247 in the faeces and biological samples of the treated subject.**

| **Sample** | **Lactobacilli** | ***L. crispatus* M247 (CFU/g)** |
|---|---|---|
| Faces at time T7 (CFU/g) | 6.10E+08 | 4.60E+07 |
| Faces at time T14 (CFU/g) | 2.60E+09 | 7.50E+08 |
| Biopsy n. 1 at time T14 | 3.00E+04 | 7.00E+03 |
| Biopsy n. 2 at time T14 | 1.80E+04 | 5.00E+03 |
| Biopsy n. 2 at time T14 | 1.20E+06 | 1.00E+05 |
| Biopsy n. 2 at time T14 | 1.50E+05 | N/I |

| | | |
|---|---|---|
| N/I = not identified | | |

### EXAMPLE 3

### Activation of immune system by L. crispatus M247 and L. crispatus MU5

The mice described in Example 1 which received *L. crispatus* M247 presented a significant reduction in the mucosal IL-6 level and an increase in IL-10, measured by expression of the corresponding messenger RNA.

However, the variations in the levels of mRNA for IL-6 and IL-10 in the MU5 group were not significant compared with the controls.

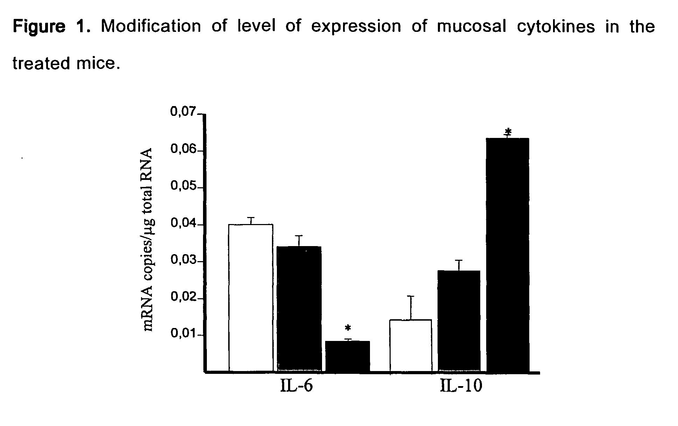

Said results are set out in Figure 1.

The white histograms refer to the controls, the grey ones to MU5 and the black ones to M247.

### EXAMPLE 4

### Activation of toll-like receptors (TLRs)

Group M247 described in Example 1 presented significantly higher TLR2 expression levels than the other groups for both the colon mucosa and isolated epithelial cells, measured by expression of the corresponding messenger RNA.

However, the MU5 group did not present significant levels of expression of TLR2 and TLR4.

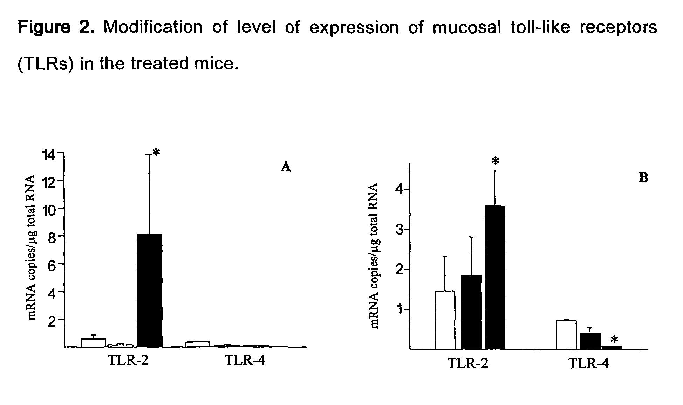

Said results are shown in Figure 2 (A and B), where the white histograms refer to the controls, the grey ones to MU5 and the black ones to M247.

Figure 2A shows the values for the colon mucosa.

Figure 2B shows the values for isolated epithelial cells.

The intestinal mucosa receptors play a crucial part in regulating various activities essential to the correct operation of the intestine. Recognition of probiotic bacteria cells by these receptors is consequently very important from the physiological standpoint.

Regulation of the TLRs is very important to protect the mucosal cells against pathogens and to repair damage.

Excessive stimulation of the TLRs can be harmful, giving rise to chronic inflammatory states.

Strain *L. crispatus* M247 has proved able to stimulate receptor TLR2, but not receptor TLR4.

Stimulation of expression of receptor TLR2 causes the dendritic cells to mature, protects the intestine against pathogenic bacteria, and stimulates the barrier function of the intestinal mucosa.

Reduction of expression of receptor TLR4 protects the intestine against the stimulating effects on the immune system of potentially pathogenic Gram-negative bacteria.

### EXAMPLE 5

### Activation of PPARgamma receptor in murine colon mucosa

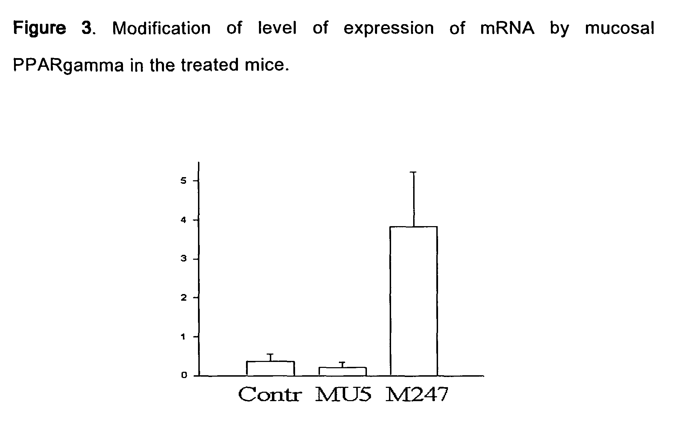

The PPARgamma receptor is crucial to the production of cytokines and chemokines in the mucosal inflammatory response. The ability of probiotic strains M247 and MU5 to stimulate the PPARgamma receptor was therefore measured by expression of the corresponding messenger RNA. The levels of expression of messenger RNA for the PPARgamma receptor were surprisingly higher in the epithelial cells of the mice treated with M247 than those treated with MU5, as shown in Figure 3.

These results were also confirmed by immunohistochemical analysis using specific staining of the PPARgamma receptor of the mucosa of the untreated animals (controls) and those treated with the probiotic strains.

Expression of the PPARgamma receptor was much higher in the epithelial layer of the mucosa of the mice treated with M247 than those treated with MU5.

### EXAMPLE 6

### Activation of PPARgamma receptor in murine epithelial cells

To verify the response of the murine epithelial cells on contact with probiotic strains M247 and MU5, the level of expression of the PPARgamma receptor was evaluated in CMT-93 cell lines following interaction with said bacterial strains.

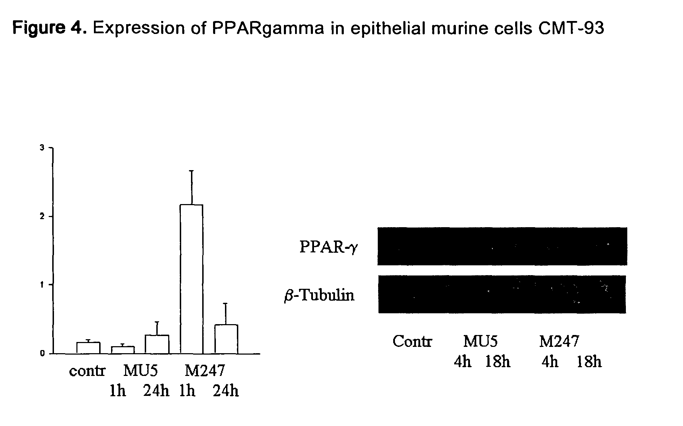

Figure 4 shows the evident difference between the expression of the PPARgamma receptor in the cells treated with M247 and those treated with MU5. The Western blot technique also confirmed the differential expression of the receptor in the cells treated with M247, whereas positive controls with β-tubulin show no differences of expression between cells stimulated following contact with M247 or MU5.

### EXAMPLE 7

### Activation mechanism of the murine intestinal mucosa receptors by probiotic strains L. crispatus M247 and MU5

The activation mechanism of PPARgamma by strain M247 was unexpectedly found to depend on its ability to produce free radicals (ROS), which are important activators of that receptor. Strain M247 proved to be a greater hydrogen peroxide producer than strain MU5 in both qualitative and quantitative terms. The method described by Eschenbach et al. 1989 was used in the first case, and the method of Gilliland, S. E. 1969, modified in accordance with the instructions of Yap P.S. and Gilliland S.E. 2000, was used in the second case.

The quantity of free radicals accumulated in the CMT-93 epithelial cells after contact with M247 was significantly higher than that of the cells treated with MU5.

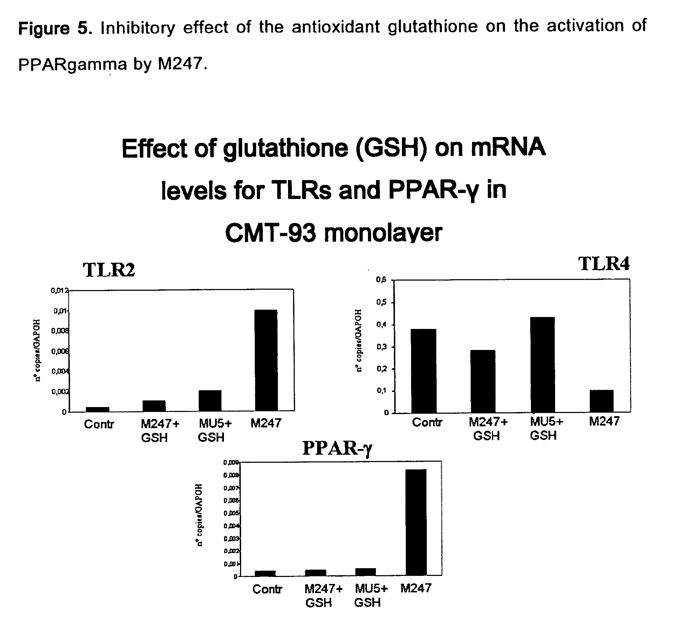

To demonstrate indirectly the activation mechanism of PPAR described above, the antioxidant agent glutathione was used to treat the epithelial cells which came into contact with probiotic M247. The presence of the antioxidant completely eliminates the effect of activation of the PPARgamma receptor generated by probiotic M247, and reduces its levels of expression in the murine mucosa (Figure 5).

### REFERENCES

Konstantinov SR, Awati A, Smidt H, Williams BA, Akkermans AD, de Vos WM (2004) Specific response of a novel and abundant Lactobacillus amylovorus-like phylotype to dietary prebiotics in the guts of weaning piglets. Applied and Environmental Microbiology 70(7): 3821-3830.

Eschenbach DA, Davick PR, Williams BL, Kleibanoff SJ, Young-Smith K, Critchlow CM, Holmes KK (1989) Prevalence of hydrogen peroxide-producing Lactobacillus species in normal women and women with bacterial vaginosis. Journal Clinical Microbiology 27:251-256.

Gilliland SE (1969) Enzymatic determination of residual hydrogen peroxide in milk. Journal Dairy Science 52: 321-324.

Yap PS, Gilliland SE (2000) Comparison of newly isolated strains of Lactobacillus delbrueckii subsp. lactis for hydrogen peroxide production at 5°C. Journal Dairy Science 83: 628-632.

## Claims

1. Strains of *Lactobacillus crispatus* able to stimulate the host immune system by producing hydrogen peroxide and consequently modulating receptor PPAR-γ.

2. Strain as claimed in claim 1, deposited under access number LMG P-23257 dated 11 April 2006 in the BCCM/LMG Bacteria Collection, Laboratorium voor Microbiologie, Ghent University, K.L. Ledeganckstraat 35, B-9000 Ghent, Belgium.

3. Probiotic compositions comprising one of the strains claimed in claim 1 or 2 as active ingredient.
